(19) Europäisches Patentamt

European Patent Office

Office européen des brevets

(11) Publication number: **0 274 267 B1**

(12) **EUROPEAN PATENT SPECIFICATION**

(45) Date of publication of patent specification: **22.07.92**  (51) Int. Cl.⁵: **A61K  7/32**

(21) Application number: **87311313.8**

(22) Date of filing: **22.12.87**

(54) **Cosmetic product.**

| | |
|---|---|
| (30) Priority: **23.12.86 GB 8630723** | (73) Proprietor: **UNILEVER PLC** **Unilever House Blackfriars P.O. Box 68** **London EC4P 4BO(GB)** |
| (43) Date of publication of application: **13.07.88 Bulletin  88/28** | (84) Designated Contracting States: **GB** |
| (45) Publication of the grant of the patent: **22.07.92 Bulletin  92/30** | (73) Proprietor: **UNILEVER NV** **Burgemeester s'Jacobplein 1 P.O. Box 760** **NL-3000 DK Rotterdam(NL)** |
| (84) Designated Contracting States: **AT BE CH DE ES FR GB GR IT LI NL SE** | (84) Designated Contracting States: **BE CH DE ES FR GR IT LI NL SE AT** |
| (56) References cited: **EP-A- 0 175 074** **DE-A- 2 503 963** **US-A- 2 087 161** **US-A- 4 137 306** **US-A- 4 524 062** | (72) Inventor: **Park, Andrew Campbell** **12 Dovepoint Road** **Great Meols Hoylake Wirral L47 6AR(GB)** |
| | (74) Representative: **van Gent, Jan Paulus et al** **Unilever N.V., Patent Division, P.O. Box 137** **NL-3130 AC Vlaardingen(NL)** |

**Description**

FIELD OF INVENTION

The invention relates to antiperspirant products, and more particularly to perfumed products comprising a finely divided antiperspirant agent suspended in a non-aqueous liquid, which when in the form of a liquid is especially suitable for dispensing from a roll-on dispenser, or from an aerosol can, and which when in the form of a solid is conveniently used in the form of a stick housed in a suitable holder or container.

BACKGROUND & PRIOR ART

It is normally considered desirable to provide antiperspirant products for topical application to human skin which are pleasantly perfumed, so as to enhance the benefit of the antiperspirant agent in not only reducing or eliminating perspiration, but also in reducing body malodour usually associated with perspiration.

To meet this need, there is, for example, a proposal in US-A-2 955 983 (Colgate-Palmolive Co) to provide an antiperspirant lotion containing aluminium chlorohydroxide, urea, ethanol, perfume and water, the water constituting about half of the formulation. In this lotion, however, the aluminium chlorohydroxide is dissolved in the water and perfuming of this type of product generally presents no problem. It is however apparent that the solution type of antiperspirant is not particularly effective in combating perspiration, owing to the impossibility of delivering to the skin surface from solution an effective amount of the antiperspirant agent to combat profuse perspiration.

EP-A-175,074 (American Cyanamid) discloses a method for producing solid gel antiperspirant compositions at a lower processing temperature, the compositions an acidic metal antiperspirant active, dibenzyl monosorbitol acetal as a gelling agent, and at least one solvent.

DE-A-2,503,963 (Thomae GMBH) discloses deodorant spray compositions containing at least 10% of one or more solvents with a boiling point of at least 37°C which evaporate on the skin, a powdered material suspended in the liquid phase, and known base, active and additive materials. The suspended powdered material is said to fix the perfume to the skin.

Attempts have been made to improve the efficacy of such solution products by providing the antiperspirant agent in a finely divided form suspended in the liquid carrier in which it is virtually insoluble, but whereas such suspension products have proved to be successful in enhancing the efficacy so far as antiperspirancy is concerned, we have noted that there is a difficulty in maintaining an adequate level of perfume stability after the product has been stored, particularly when the product contains ethanol. It is accordingly apparent that perfumes can be unstable in suspension-type alcoholic lotion antiperspirant products to such an extent that unless freshly prepared, little or no fragrance may remain detectable in such products after storage in the factory, shop or home under normal ambient temperature conditions, for more than about one month.

In our attempts to discover why this may be so and to establish conditions whereby the fragrance of perfumed anhydrous alcoholic antiperspirant products might be preserved until the product is used, we have found that the addition of one or more special perfume stabilising agents to such formulations mitigates the deterioration of the perfume, such that the product remains fragrant for many months with little or no noticeable decrease in the intensity of the fragrance present when the product is first manufactured.

We have also discovered that these same perfume stablising agents inhibit the dissolution of the antiperspirant agent in the anhydrous alcohol which forms at least part of the product, with the consequent benefit that the antiperspirant efficacy particularly in liquid lotions for use in roll-on dispensers is enhanced, since a greater quantity of the antiperspirant-agent in solid form will then be deposited on the skin.

It is also apparent that perfume deterioration is mitigated and dissolution of antiperspirant agent in anhydrous alcohol is reduced in the corresponding aerosol and stick products.

DEFINITION OF INVENTION

According to the invention, there is provided an anhydrous antiperspirant product suitable for applying to the skin which comprises:
i) from 1 to 60% by weight of a finely-divided powder antiperspirant agent;
ii) from 10 to 90% by weight of an anhydrous liquid or solidified liquid medium, comprising an anhydrous alcohol chosen from ethanol, isopropanol and mixtures thereof;
iii) from 0.001 to 10% by weight of a perfume; and

iv) from 0.1 to 25% by weight, expressed in terms of the anhydrous alcohol present, of a perfume-stabilising agent chosen from compounds having a basic nitrogen function and compounds having a basic oxygen function, and drying agents and mixtures thereof.

DISCLOSURE OF THE INVENTION

The Antiperspirant Agent

The antiperspirant product according to the invention comprises an antiperspirant agent in the form of a finely divided powder so as to enable it to be suspended when desired in the liquid medium. Preferably the antiperspirant agent powder has a maximum particle diameter of no greater than 100$\mu$m preferably no greater than 70$\mu$m and preferably about 45$\mu$m.

Examples of suitable antiperspirant agents include aluminium chloride, aluminium sulphate, aluminium chlorohydrate, basic aluminium bromide, zirconyl chloride; zirconyl hydroxide; zirconyl chlorohydrate complexes of aluminium hydroxide, zirconyl chloride and aluminium chlorohydrate; complexes of aluminium hydroxide, zirconyl chlorohydrate, and aluminium chlorohydrate; complexes of dihydroxyaluminium glycinate, zirconyl chloride and/or zirconyl chlorohydrate and aluminium chlorohydrate; complexes of zirconyl chloride and/or zirconyl clhorohydrate and aluminium chlorohydrate; complexes of zirconyl chloride and/or zirconyl chlorohydrate with aluminium chlorohydrate and an amino acid, such as glycine; and mixtures of two or more of the above. Most preferred materials are those which have a high antiperspirant efficacy. One class of such materials are the aluminium zirconium chlorohydrate complexes. Examples are aluminium zirconium trichlorohydrate, aluminium zirconium tetrachlorohydrate and aluminium zirconium pentachlorohydrate (these are CTFA generic names). These compounds may be combined with glycine to give for example the compounds known under the CTFA generic names aluminium zirconium trichlorohydrex-GLY and aluminium zirconium tetrachlorohydrex-GLY. Suitable aluminium zirconium chlrohydrate powders for use in the antiperspirant compositions of this invention are available from the Reheis Chemical Company under the trade names REZAL 36GP and REZAL 67P (REZAL is trade mark) and from Wickhen Products, Incorporated under the trade names WICKENOL 369 and WICKENOL 379 and WICKENOL 373 (WICKENOL is a trade mark). Other preferred antiperspirant active materials of high efficacy are the special active forms of basic aluminium chloride which have a particular distribution of polymeric species in aqueous solution and obtainable by procedures described in US Patent No. 4 359 456 (Gosling et al). Similar processes for making highly active forms of aluminium chlorohydrate involving the ageing of aluminium chlorohydrate in an aqueous medium are described in British Patent Specification No 2 048 229 (Gillette). Other suitable materials are described in British Patent Specification No. 2 144 992 (Gillette). The antiperspirant agent may also be a urea or glycine complex of aluminium chlorohydrate prepared as described in European Patent No. 6738 (Unilever) and British Patent No. 1 597 497 (Unilever), respectively.

A further example of activated aluminium chlorohydrate which can be employed as the antiperspirant agent is REACH available from Reheis.

The amount of the antiperspirant agent present in the product according to the invention will depend on the form of the product and in general will be within the range of from 1 to 60%, preferably from 5 to 50% by weight. When the product is a propellant-based aerosol, these amounts refer to the aerosol concentrate without the propellant.

The anhydrous liquid or solidified liquid medium

The antiperspirant product according to the invention also comprises an anhydrous medium comprising an alcohol chosen from anhydrous ethanol, anhydrous isopropanol and mixtures thereof. By "anhydrous" is meant that the ethanol and/or the isopropanol contains no more than 1% by weight of water.

The anhydrous liquid or solidified liquid medium preferably comprises the anhydrous alcohol.

It is, however, possible for other anhydrous liquids to form part of the anhydrous medium of the antiperspirant product of the invention. Such additional liquids can be incorporated to give emollient cosmetic benefits and can be either hydrophilic or hydrophobic liquids.

Examples of the emollient liquids than can optionally be employed include a polar liquid such as water-miscible polyoxyalkylene glycol or a water-miscible partial butyl either thereof; hexylene glycol; a $C_1$-$C_4$ alkyl monoether or a simple or condensed $C_2$-$C_4$ alkylene glycol for example dipropylene glycol monomethyl ether; or 2-ethyl-1,3-hexane diol. Other commercially available hydrophilic materials which are suitable are Pluronics (eg. Poloxamer 101, Poloxamer 105, Poloxamer 181, Poloxamer 182), Carbowaxes

3

(eg. PEG-4, PEG-8, PEG-12), Witconol APEM (PPG-3 Myreth-3), Witconol APES (PPG-9 Steareth-3), Standamul OXL (PPG-10 Cetearth-20), Procetyl AWS Modified (PPG-8 Ceteth-2). Glycols and their citrate, lactate and tartrate esters as taught in Canadian Patents 1 121 728; 1 121 729 and 1 121 730 can also be used. Hydrophilic materials which are the dimethicone copolyols that is polymers of dimethylsiloxane with polyoxyethylene and/or polyoxypropylene side chains. Examples of these are SILWET L-270, L-7600 and L-7610 from Union Carbide, Silicone 190 and 193 surfactants both from Dow Corning and ABIL B 8842, 8843 and 8851 from Goldschmidt. These dimethicone copolyols are also referred to in the literature as polyalkylene oxide modified dimethylpolysiloxanes, as silicone glycol copolymers and as polysilicone polyether copolymers. Dimethyl isosorbide is a further example of a suitable hydrophilic liquid.

Examples of non-polar water-immiscible liquids are the linear volatile silicones having 2 to 9 silicon atoms such as hexamethyl disiloxane, the cyclic volatile silicones having 3 to 6 silicon atoms such as tetramer and pentamer, linear non-volatile silicones, paraffin oils, fatty acid esters such as isopropyl myristate and dibutyl phthalate, and polyoxypropylene fatty ethers such as Fluid AP, and mixtures thereof.

When the product according to the invention is solid, for example in the form of cosmetic stick, then the solidfied liquid medium can comprise a gelling agent.

Examples of gelling agents include low melting point waxes, having a melting point of from about 40°C to 70°C, such as fatty acids and fatty alcohols containing from 8 to 22 carbon atoms, silicone waxes and glycerol monostearate. Preferred examples of such waxes include lauric acid, stearic acid, palmitic acid, cetyl alcohol, stearyl alcohol, myristyl alcohol and behenyl alcohol. The amount of the low melting point wax when employed will normally be from 8 to 35%, preferably 12 to 25% by weight of the product.

A quantity of water-insoluble high melting wax may also be incorporated in the antiperspirant stick composition to help provide the basic structure of the stick. Suitable waxes for this purpose are water-insoluble waxes, having a melting point of from about 70°C to about 100°C. Examples of suitable waxes include;

Castorwax MP80, Synchrowax HRC, carnaubau, beezwax spermaceti, ozokerite and paraffin wax. These may constitute from 0 to 10%, preferably from 2 to 8% by weight of the product.

The normally solid higher fatty acid amides of alkylolamines are also useful as gelling agents in the antiperspirant stick compositions of the invention. Such amides can be derived by the usual condensation, at elevated temperatures of from 150 to 175°C of normally solid higher fatty acids such as palmitic acid, stearic acid, myristic acid and lauric acid, with primary or secondary alkylolamines or hydroxyalkyl amines such as, monoethanoloamine, diethanolamine, n-propanolamine, hydroxyethyl ethylenediamine, glycerolamine, 1-amino-2, 3-propanediol and 2-amino-1,3-propanediol. The condensation products of myristic acid, palmitic acid or stearic acid with monoethanolamine are preferred. The alkylolamides when employed should constitute from 15 to 35% preferably from 20 to 30% by weight of the product.

The gelling agent may also be dibenzyl monosorbitol acetal (DBS), desirably together with a co-solvent such as dihydric and polyhydric alcohols and stabilising agents to prevent acid decomposition of the acetal and subsequent gel breakdown. When employed, DBS should be present in an amount of from 0.5 to 10% preferably 1 to 5% by weight of the product.

Specific examples of co-solvents for DBS include 2-methoxyethanol, 2-ethoxyethanol, ethylene glycol, diethylene glycol, diethylene glycol monomethylether, 1,2-propylene glycol, 1,3-butylene glycol, dipropylene glycol, hexylene glycol, 2,4-dihydroxy-2-methylpentane, polyetherlene glycols and mixtures thereof. These should be present in amounts of from 10 to 60%, preferably from 15 to 45% by weight of the product.

Agents suitable for stabilising products according to the invention containing dibenzyl monosorbitol acetal include amines such as methenamine (hexamethylene tetramine), amides such as acetamide monoethanolamide and cocofatty acid monoethanolamide and basic metallic salts such as zinc oxide, zinc acetate, calcium acetate, zinc carbonate, potassium carbonate, magnesium carbonate and calcium hydroxide. Mixtures of the above organic and inorganic gel stabilising agents are also effective. Ordinarily, the stabilisers will be used in an amount ranging from 0.02 to 10%, preferably from 0.1 to 5% by weight of the product.

The amount of the anhydrous liquid or solidified liquid medium present in the product according to the invention will usually form from 10 to 90%, preferably from 30 to 80% by weight, the anhydrous alcohol forming at least 10% by weight of the product. The alcohol can alternatively comprise from 10 to 80%, preferably from 15 to 75% by weight expressed in terms of the antiperspirant product.

The perfume

The antiperspirant product according to the invention also comprises a perfume, the function of which is primarily to enhance the benefits of the product when applied topically to skin to reduce or eliminate perspiration. The fragrance derived from the product can thus assist in masking body malodour frequently associated with perspiration.

Although the nature and formulation of the perfume is not critical, it is preferred to employ a perfume having distinct deodorant properties which functions not only as an odour maskant, but also in the elimination of materials accummulating at the skin surface which contribute to body malodour. Deodorant perfumes of this type are described, for example, in US patent 4 278 658 (Lever Brothers Company).

The amount of perfume present in the product according to the invention will usually form from 0.001 to 10%, preferably from 0.1 to 5% and ideally from 0.2 to 2% by weight.

The perfume stabilising agent

The antiperspirant product according to the invention also comprises a perfume stabilising agent.

As has been stated earlier, the perfume normally included in suspension-type lotion antiperspirant products can deteriorate rapidly during storage prior to use. The reasons for this are not fully understood, particularly since solution type antiperspirant compositions apparently do not suffer from the same problem. It does appear, however, that the loss of fragrance and, where applicable, the deodorant properties of a perfume in the suspension type products such as the antiperspirant compositions according to the invention can be mitigated by including in the product an effective amount of a perfume stabilising agent chosen from compounds having a basic nitrogen function, compounds having a basic oxygen function and drying agents, or mixtures thereof.

Examples of compounds having a basic nitrogen function are urea; natural amino acids for example glycine, alanine, taurine, serine, sarcosine, arginine valine, leucine, proline, methionine, threonine, ornithine, histidine, lysine monohydrochloride, glutamic acid monomethyl ester (which amino acids include the neutral amino acids and basic amino acids); and organic amines, such as $C_1$ to $C_{20}$ alkylamines and hydroxylamines, for example triethylamine, mixed $C_{13}/C_{15}$ alkylamine and 2-amino-2-methyl-propan-1-ol.

Examples of compounds having a basic oxygen function are inorganic bases such as the hydroxides of the alkali metal and the alkaline-earth metals and ammonium hydroxide, including sodium hydroxide, potassium hydroxide, lithium hydroxide, calcium hydroxide and magnesium hydroxide, as well as the basic metallic oxides, for example aluminium oxide, zinc oxide and lanthanum oxide; and the alkali metal salts of inorganic acids, such as sodium carbonate, sodium thiosulphate, anhydrous sodium sulphate, dipotassium hydrogen orthophosphate, zinc carbonate and $C_1$ to $C_{20}$ organic carboxylic acids for example trisodium citrate, sodium acetate sodium n-octanoate and sodium stearate. Drying agents such as molecular sieve Type 3A can also be used as a perfume stabilising agent.

The amount of the perfume stabilising agent present in the product according to the invention is that which is effective in stabilising the perfume in the composition for at least one month at a storage temperature of 37°C. The actual amount required will depend on the particular perfume stabiliser employed, and is conveniently related to the amount of the anhydrous alcohol present in the product. In general, a sufficient amount will be from about 0.1 to about 25% by weight of the anhydrous alcohol present in the liquid medium of the product. The amount of urea or amino acid will generally be in the range of from 0.5 to 25% preferably from 1 to 10% and ideally from 1 to 5% by weight of the alcohol, although in practice any amount can be included which is consistent with an acceptable product. The organic amines can be used in an amount of from 0.5 to 4% by weight of the alcohol. The alkali metal inorganic bases and ammonium hydroxide are suitably used in amounts of about 0.1 to about 2% by weight of the alcohol, and other inorganic bases, basic oxides and inorganic acid salts in amounts of about 0.5 to 10% by weight of the alcohol. The salts of the $C_1$-$C_{20}$ carboxylic acids and the drying agent molecular sieve type 3A can be used in an amount of from 0.5 to 20% by weight of the alcohol.

OPTIONAL INGREDIENTS

The product according to the invention can also comprise optional ingredients further to improve and modify its performance and acceptability as judged by the consumer. Examples of such optional ingredients include suspending agents and colourants.

Optional suspending agent

The antiperspirant product according to the invention can also optionally comprise a hydrophobic clay as an agent for suspending the particles of the antiperspirant in the anhydrous liquid or solidified liquid medium.

Particularly suitable clays are the hydrophobically treated smectite clays. Smectite clays are those characterised by having an expanding lattice. Examples of these clays include the montmorillonites, hectorites, and colloidal magnesium aluminium silicates.

Montmorillonite is colloidal, hydrated aluminium silicate obtained from bentonite of which it is the predominant constituent. A detailed discussion of bentonites can be found in the Kirk-Othmer Encyclopedia of Chemical Technology, 2nd Edition, Vol. 3 (1964) pp. 339-360, published by Interscience Publishers.

Hectorite, also a smectite clay, differs from montmorillonite in that there is almost a complete substitution of aluminium in the lattice structure of montmorillonite by magnesium and in addition, it contains lithium and fluorine.

The magnesium aluminium silicates are complexes of colloidal magnesium aluminium silicate richer in magnesium than aluminium. The hydrophobically treated magnesium aluminium silicates are commercially available under the name VEEGUM PRO from the R T Vanderbilt Co.

Preferred suspending agents for optional use in the invention are hydrophobic clays available under the trade name of BENTONE. BENTONES are prepared by reacting a suitable clay in a cation exchange system with an amine. Different amines are reacted to obtain a variety of BENTONES, which may also differ in proportions of Si, MgO and $Al_2O_3$. Examples of useful BENTONE suspending agents are BENTONE-27, which is a stearaluminium hectorite; BENTONE-34, which is quaternium 18 bentonite; BENTONE-38, which is quaternium 18 hectorite; and BENTONE-14 which is a clay-extended quaternium 18 hectorite, all of which have a particle size of below 5 microns and are commercially available in USA from NL Industries Inc. Other suitable BENTONE clays are BENTONE SD1 and Bentone SD2.

Yet further suitable clays are those hydrophobically treated smectite clays available under the trade names PERCHEM and TIXOGEL. Specific examples are PERCHEM clays 44, 97 and 108 and TIXOGEL VZ.

A further class of hydrophobically treated clays comprises hormite clays, an example of which is hydrophobically modified attapulgite clay available under the name PERCHEM DMA. PERCHEM clays are sold by Perchem Limited of Harlow, Essex, Great Britain and TIXOGEL clays by Production Chemicals Limited of Stockport, Cheshire, Great Britain.

Other useful suspending agents are fine silica powders especially the fumed silicas available commercially under the names AEROSIL and CAB-O-SIL, and hydroxypropyl cellulose (KLUCEL).

Mixtures of suspending agent can also be employed.

The amount of the suspending agent which is optionally present in the product according to the invention will in general be within the range of from 0.5 to 15% by weight.

## PROCESS FOR MANUFACTURE OF ANTIPERSPIRANT COMPOSITION

The invention also provides a process for the manufacture of the antiperspirant product according to the invention, which process comprises the step of mixing together the antiperspirant agent, the anhydrous liquid, the perfume and the perfume stabilising agent. It is preferable to add the perfume-stabilising agent to the anhydrous alcohol either before or simultaneously with the perfume, but not after it. The product so obtained is then filled into a convenient dispenser such as a roll-on dispenser, an aerosol can, or a mould, when the product is to be solidified.

According to a preferred process, a thickening agent is also incorporated into the product so as to suspend the antiperspirant agent in the form of fine particles.

## METHOD OF USE

The invention also relates to a method for the control of perspiration which comprises the step of applying the product according to the invention in the form of a lotion from a roll-on dispenser a spray from an aerosol can or a solidified stick to the skin, particularly to the underarm, in order to prevent or reduce perspiration.

## EVIDENCE TO CONFIRM PERFUME STABILITY

The stability during storage of perfume in the suspension type antiperspirant product according to the invention is assessed by a standard test in which antiperspirant products, either with or without a perfume-stabilising agent, are compared using a subjective olfactory method by two or more expert assessors.

In order to perform this test, a lotion containing an antiperspirant agent, together with anhydrous ethanol and/or anhydrous isopropanol and a perfume, as well as other materials as desired with the exception of

6

EP 0 274 267 B1

perfume-stabilising agents is prepared.

A perfume-stabilising agent under test is then introduced into a part of the lotion so prepared to provide the test sample, the remaining part of the lotion containing no perfume-stabilising agent serving as the control.

Portions of the test and control lotion samples so prepared are stored in sealed containers at 37°C for between one and three months, and another portion of both test and control samples is also stored at 0°C over the same time period.

It has been established over many years that perfumes require time to "settle down" in an antiperspirant formulation. Storage at 0°C has the effect of allowing "settling down" to take place yet avoiding further perfume modification. Hence by comparing the same perfumed lotion stored at 0°C and 37°C for the same time, it is possible to establish the extent to which the perfume stored at the higher temperature has been modified over and above the usually small change in note brought about by simply putting the perfume into the formulation.

After storage, the extent of perfume modification was estimated using a 0 to 5 hedonic odour scale as follows:

    0     : unmodified
    1     : v. slight modification
    2     : slight modification
    3     : moderate modification
    4     : severe modification
    5     : unacceptable

Scores of 2.5 or less were considered to represent an acceptable degree of perfume modification.

By way of illustrating the invention, Table 1 shows the effect of adding urea (5.4& w/w of ethanol) to a range of perfumes contained in lotions having ethanol as the liquid medium and aluminium zirconium tetrachlorohydrex -GLY (ZAG) or a special active form of aluminium chloride (AACH) as antiperspirant actives. With all systems, urea has the effect of increasing perfume stability and for most perfumes the degree of modification changes from being unacceptable to being acceptable in presence of urea.

A further illustration is given in Table 2 in which it is shown that a number of additives having a basic oxygen or a basic nitrogen functional group can stabilise perfumes via a lotion containing ethanol as the liquid medium and REZAL 36GP as the antiperspirant active. A drying agent, Molecular Sieve Type 3A, is also seen (Table 2) to be effective.

Table 3 shows the effect of adding urea (5% w/w of ethanol) to a REZAL 36GP/ethanol lotion to which a range of individual perfume materials have been added rather than complete perfumes. With only one material (methyl cinnamic aldehyde) did urea reduce odour stability. As for the other 15 perfume materials, urea improved the odour stability of 9 of these and had no influence on the remainder.

It should be noted that some of the trade names use in this specification, such as Rezal, Wickenol, Reach, Poloxamer, Silwet, Abil, Betone, Tixogel, Perchem, Aerosil, Cab-o-sil, Klucel, and others may be Registered Trade Markes in some of the Contracting States.

7

TABLE 1

Effect of added urea on stability of seven perfumes after 6 weeks storage
at 37°C in lotions having ZAG and AACH as antiperspirant agents
and ethanol as the liquid medium

ODOUR   SCORE

| PERFUME CODE | PD726 | PD667 | ST177 | PD725 | PD650 | HD400A | ST177F |
|---|---|---|---|---|---|---|---|
| ZAG without urea | 2.4 | 3.4 | 4.0 | 3.6 | 4.6 | 4.8 | 3.4 |
| ZAG with urea | 1.2 | 2.2 | 1.0 | 1.2 | 2.2 | 2.4 | 2.2 |
| AACH without urea | 3.4 | 3.4 | 4.0 | 3.4 | 4.6 | 3.6 | 3.2 |
| AACH with urea | 1.4 | 1.2 | 2.4 | 1.4 | 3.4 | 2.4 | 2.6 |

## TABLE 2

### Effect of Various Additives on the Stability of Two Commercially Available Perfumes after 1 month's Storage at 37°C in a Lotion having REZAL 36GP as Antiperspirant Agent and Ethanol as the Liquid Medium

| | | ODOUR SCORE | |
| --- | --- | --- | --- |
| Additive | Perfume: | HD400 | TS849 |
| Molecular Sieve Type 3A (5%) | | 1.0 | 3.0 |
| Sodium Stearate (5%) | | 2.0 | 2.0 |
| Trisodium citrate (5%) | | 0.0 | 0.0 |
| Zinc oxide (5%) | | 0.8 | 1.0 |
| Dipotassium hydrogen orthophosphate (5%) | | 1.0 | 3.5 |
| Calcium hydroxide (1%) | | 0.5 | 0.0 |
| Urea (5%) | | 2.0 | 1.0 |
| Triethylamine (2%) | | 1.0 | 1.0 |
| Amminomethylpropanol (3%) | | 1.0 | 0.5 |
| Glycine (5%) | | 1.0 | 0.3 |
| No additive | | 3.0 | 4.0 |

[Note: the ( %) values placed after the additives is with respect to the total product.

9

## TABLE 3

### Effect of Added Urea on Stability of Individual Perfume Components after 13 weeks Storage at 37°C in a Lotion having REZAL 36GP as Antiperspirant Agent and Ethanol as the Liquid Medium

| | Odour Score | |
|---|---|---|
| Perfume Component | Without Urea | With Urea |
| Ethyl salicylate | 2.5 | 1.5 |
| Pinene | 4.0 | 3.0 |
| Limonene DQ | 5.0 | 3.0 |
| Citronellol STD 95% | 0.0 | 0.0 |
| Lilial | 2.0 | 1.0 |
| Methyl heptenone | 2.0 | 1.0 |
| Acetophenone | 0.0 | 0.0 |
| Eugenol | 0.0 | 0.0 |
| Ethyl benzoate | 0.0 | 0.0 |
| Geraniol | 5.0 | 3.0 |
| Ionone | 5.0 | 0.0 |
| Benzaldehyde | 3.0 | 1.0 |
| Pentan-2-one | 0.0 | 0.0 |
| Methyl cinnamic aldehyde | 0.0 | 1.0 |
| Methyl cinnamate | 0.0 | 0.0 |
| Aldehyde L9 | 2.0 | 0.0 |

In a further test to confirm perfume stability, test and control roll-on formulations (with and without added urea) were prepared as follows:

|  | % w/w | |
| --- | --- | --- |
|  | Test | Control |
| REZAL 36GP | 25 | 25 |
| BENTONE 38 | 10 | 10 |
| Anhydrous alcohol | 60.8 | 64 |
| Urea | 3.2 * | - |
| Deodorant perfume | 1 | 1 |

* as a 5% by weight solution in ethanol.

After 10 weeks storage, each of the above formulations for each of 5 different perfumes was evaluated by a panel of trained assessors as previously described.

The results of this assessment are summarised in Table 4 below:

## TABLE   4

| Deodorant Perfume | with(+)without(-) added urea | Mean odour score |
| --- | --- | --- |
| A2 | + | 1 |
|  | - | 3 |
| B3 | + | 1 |
|  | - | 4 |
| C4 | + | 0 |
|  | - | 2 |
| D5 | + | 0 |
|  | - | 2 |
| E6 | + | 2 |
|  | - | 4 |

It was concluded from the above results that the presence of urea in all cases stabilised the deodorant perfume, whereas in the absence of urea, moderate or severe modification to the deodorant perfume occured.

The deodorant perfume formulations employed in this test were as follows:

Deodorant Perfume A2

| | Parts |
|---|---|
| 6-Acetyl-1,1,3,4,4,6-hexamethyl-tetrahydro naphthalate | 3.00 |
| Bergamot base 37 | 20.00 |
| Carvacrol | 3.50 |
| Citronellyl acetate | 5.00 |
| Dipropylene glycol | 4.75 |
| Geranyl nitrile | 1.50 |
| Indole | 1.00 |
| Lemongrass oil | 3.00 |
| Lime AB 402 | 10.00 |
| Lavandin oil | 4.00 |
| l-Menthol | 8.00 |
| 3a-Methyl-dodecahydro-6,6,9a-trimethyl naptho-2(2,1-b)-furan | 0.25 |
| β-Methyl naphthyl ketone | 5.00 |
| β-Naphthol methyl ether | 9.00 |
| Neroli base 78 | 6.00 |
| Pomeransol AB 314 | 6.00 |
| Petitgrain oil (terpeneless) | 4.00 |
| Orange oil sweet | 5.00 |
| Thyme oil red | 1.00 |
| | 100.00 |

## Deodorant Perfume B3

|  | Parts |
| --- | --- |
| p-t-Amylcyclohexanone | 5.00 |
| Benzoin Siam resinoid | 5.00 |
| Bergamot AB 430 | 15.00 |
| Coumarin | 4.00 |
| Diethyl phthalate | 4.35 |
| Geranium oil | 5.00 |
| Hercolyn D | 12.25 |
| 1,3,4,6,7,8-Hexahydro-4,6,6,7,8,8-hexamethyl cyclopenta-$\gamma$-2-benzopyran | 3.00 |
| Lavandin oil | 10.00 |
| $\alpha$-iso-Methyl ionone | 12.00 |
| Mousse de Chene Yugo | 1.25 |
| Musk ambrette | 3.00 |
| Pimento leaf oil | 10.00 |
| Rosenta AB 380 | 10.00 |
| Rose-D-oxide | 0.15 |
|  | 100.00 |

## Deodorant Perfume C4

|  | Parts |
|---|---:|
| Bergamot AB 430 | 8.00 |
| p-t-Butylcyclohexyl acetate | 4.30 |
| Citronella oil | 6.00 |
| Diethyl phthalate | 8.25 |
| Ethyl vanillin | 0.20 |
| iso-Eugenol | 5.00 |
| Green Herbal AB 502 | 15.00 |
| 2-n-Heptylcyclopentanone | 0.50 |
| Indole | 1.50 |
| Inonyl formate | 5.00 |
| LRG 201 | 1.25 |
| α-iso-Methyl ionone | 5.00 |
| β-Naphthol methylether | 7.50 |
| Nonanediol-1:3-diacetate | 4.00 |
| Patchouli oil | 7.00 |
| Phenylethyl phenyl acetate | 5.00 |
| Rosenta AB 380 | 6.00 |
| Sandalone | 4.00 |
| Tetrahydro muguol | 6.00 |
| γ-Undecalactone | 0.50 |
|  | 100.00 |

14

## Deodorant Perfume D5

| | Parts |
|---|---|
| 6-Acetyl-1,1,3,4,4,6-Hexamethyl tetra- hydro napththalate | 2.50 |
| p-t-Amylcyclohexanone | 0.06 |
| Benzyl salicylate | 15.00 |
| Bergamot AB 430 | 15.00 |
| Cinnamic alcohol | 5.00 |
| Diethyl phthalate | 8.04 |
| Dimethyl benzyl carbinyl acetate | 2.50 |
| Dimyrcetol | 16.00 |
| Dipropylene glycol | 14.25 |
| Geraniol | 5.00 |
| Isobutyl phenyl acetate | 5.00 |
| 3a-Methyl-dodecahydro-6,6,9a-trimethyl- naphtho-2(2,1-b) furan | 0.75 |
| Methyl salicylate | 0.50 |
| Mousse de Chene Yugo | 6.00 |
| Nonanolide-1:4 | 0.20 |
| Pelargene | 4.00 |
| Trichloromethyl phenyl carbinyl acetate | 0.20 |
| | 100.00 |

## Deodorant Perfume E6

|                                                                   | Parts  |
|-------------------------------------------------------------------|--------|
| Benzyl propionate                                                 | 4.00   |
| Bergamot oil                                                      | 15.00  |
| o-t-Butylcyclohexyl acetate                                       | 2.00   |
| p-t-Butyl-α-methyl hydrocinnamic aldehyde                         | 15.00  |
| Clove leaf oil                                                    | 10.00  |
| Diethyl phthalate                                                 | 9.25   |
| Dimethyl benzyl carbinyl acetate                                  | 5.00   |
| Inonyl acetate                                                    | 10.00  |
| iso-Butyl benzoate                                                | 5.00   |
| LRG-201                                                           | 1.25   |
| 3a-Methyl-dodecahydro-6,6,9a-trimethyl-<br>naphtho-2 (2,1-b)-furan | 0.50   |
| Neroli oil                                                        | 3.00   |
| Petitgrain oil                                                    | 10.00  |
| Phenyl ethyl alcohol                                              | 10.00  |
|                                                                   | 100.00 |

## EVIDENCE TO CONFIRM REDUCTION IN SOLUBILITY OF ANTIPERSPIRANT AGENT IN ETHANOL

The following experiments illustrate the ability of the perfume stablising agents to inhibit the dissolution in anhydrous alcohol of antiperspirant agents. Percentages are by weight.

## Experiment 1

To saturated solutions of urea in anhydrous ethanol (urea concentration about 5.4% w/w) were added various antiperspirant agents in an amount of 25% w/w. The compositions were mixed for 48 hours at 20°C. The concentration of aluminium in the liquid phase was then determined by analysis using atomic absorption spectroscopy. Such concentrations are expressed herein as M which stands for moles of aluminium per litre of ethanolic solution. The anhydrous ethanol was a grade of absolute alcohol containing a maximum amount of water of 0.3% by weight.

The results for various antiperspirant agents are given in Table 5.

16

## TABLE 5

| Active | M | |
|---|---|---|
| | No urea | Urea |
| Aluminium chlorohydrate | 1.374 | 0.083 |
| Aluminium zirconium tetra chlorohydrate glycine complex[1] | 0.011 | 0.001 |
| Aluminium zirconium pentachlorohydrate[2] | 1.637 | 0.004 |
| Aluminium zirconium trichlorohydrate[3] | 1.376 | 0.011 |
| Basic aluminium bromide [4] | 1.844 | 0.715 |
| Aluminium chlorohydrate urea complex [5] | 0.030 | 0.001 |

1 - REZAL 36GP

2 - REZAL 67P

3 - REZAL 36P

4 - 2 hour test period

5 - according to European Patent No. 6738

For all the antiperspirant agents, the presence of the urea effected a substantial reduction in the amount of the agent passing into solution in the alcohol.

Experiment 2

In this experiment the effect of varying the amount of urea on the extent of dissolution in anhydrous ethanol of two antiperspirant agents is illustrated in Table 6.

## TABLE 6

| Amount of Urea % (w/w) | Aluminium chlorohydrate | $\underline{M}$ Aluminium zirconium trichlorohydrate |
|---|---|---|
| 0 | 1.374 | 1.376 |
| 1 | 0.617 | 0.128 |
| 3 | 0.259 | 0.067 |
| 4 | 0.180 | 0.013 |
| 5.4 | 0.083 | 0.011 |
| 10.8 | 0.002 | 0.004 |

Experiment 3

In this experiment the effect of additives other than urea in inhibiting the dissolution of aluminium chlorohydrate in anhydrous ethanol was demonstrated, the results being summarised in Table 7.

## TABLE 7

| Additive | Amount of Additive % (w/w) | M |
|---|---|---|
| Glycine | 5 | 0.015 |
| Leucine | 5 | 0.012 |
| L-Histidine | 5 | 0.002 |
| L-Lysine hydrochloride | 5 | 0.015 |
| L-Arginine | 5 | 0.002 |
| Triethylamine | 2 | 0.001 |
| Mixed $C_{13}/C_{15}$ amine | 2 | 0.009 |
| 2-Amino-2-methyl propan-1-ol | 3 | 0.012 |
| Sodium hydroxide | 1 | 0.001 |
| Calcium hydroxide | 1 | 0.003 |
| Ammonium hydroxide | 1 | 0.022 |
| Zinc oxide | 5 | 0.001 |
| Sodium acetate | 5 | 0.006 |
| Trisodium citrate | 5 | 0.003 |
| Sodium n-octanoate | 5 | 0.095 |
| Sodium stearate | 5 | 0.042 |
| Sodium carbonate | 2 | 0.178 |
| Sodium thiosulphate | 5 | 0.276 |
| Sodium tetraborate | 5 | 0.013 |
| Dipotassium hydrogen orthophosphate | 5 | 0.159 |
| Sodium sulphate (anhydrous) | 5 | 0.666 |
| Molecular Sieve Type 3A | 5 | 0.003 |
| No additive | - | 1.374 |

Experiment 4

In this experiment the effect of additives other than urea in inhibiting the dissolution of aluminium zirconium trichlorohydrate in anhydrous ethanol was shown, the results being given in Table 8.

## TABLE 8

| Additive | Amount of Additive % (w/w) | M |
|---|---|---|
| Glycine | 5 | 0.001 |
| Leucine | 5 | 0.264 |
| Triethylamine | 2 | 0.008 |
| Mixed $C_{13}/C_{15}$ amine | 2 | 0.756 |
| 2-amino-2-methyl-propan-1-ol | 3 | 0.025 |
| Sodium hydroxide | 1 | 0.078 |
| Calcium hydroxide | 5 | 0.001 |
| Ammonium hydroxide | 5 | 0.007 |
| Sodium acetate | 5 | 0.092 |
| Trisodium citrate | 5 | 0.001 |
| Sodium sulphate (anhydrous) | 5 | 0.492 |
| Molecular Sieve Type 3A | 5 | 0.138 |
| No additive | - | 1.376 |

Experiment 5

In this experiment, the effect of additives in inhibiting the dissolution of aluminum zirconium trichlorohydrate in anhydrous isopropanol was shown, the results being given in Table 9.

## TABLE 9

| Additive | Amount of Additive % (w/w) | M |
|---|---|---|
| Urea | 2 | 0.008 |
| Glycine | 5 | 0.000 |
| L-Arginine | 5 | 0.003 |
| L-Aspartic acid[1] | 5 | 0.324 |
| Calcium hydroxide | 5 | 0.000 |
| Mixed $C_{13}/C_{15}$ amine | 2 | 0.003 |
| 2-amino-2-methyl propan-1-ol | 2 | 0.001 |
| Sodium acetate | 5 | 0.001 |
| Molecular Sieve Type 3A | 5 | 0.000 |
| No additive | - | 0.018 |

1 - a comparative test showing no inhibitive effect when using an acidic amino acid

Experiment 6

Various other compounds that have been shown to be ineffective for inhibiting the dissolution of aluminum chlorohydrate in anhydrous ethanol are referred to in Table 10.

21

## TABLE 10

| Additive | % (w/w) | M |
|---|---|---|
| Acetamide | 3 | 2.052 |
| Coconut diethanolamide | 3 | 1.175 |
| Cetyl trimethylammonium bromide | 5 | 2.230 |
| Ammonium thiocyanate | 5 | 1.550 |
| Sodium chloride | 2 | 1.267 |
| Sodium bromide | 2 | 1.550 |
| Sodium iodide | 5 | 1.757 |
| Citric acid | 5 | 2.323 |
| Isopropyl myristate | 10 | 1.256 |
| Cyclic volatile silicone | 10 | 1.111 |
| Dipropylene glycol methyl ether | 10 | 1.215 |
| Butylene glycol | 10 | 1.674 |
| No additive | - | 1.374 |

EXAMPLES

The following examples illustrate the invention.

%

| Examples: | 1 | 2 | 3 | 4 | 5 | 6 |
|---|---|---|---|---|---|---|
| AACH[1] | 25.0 | 25.0 | 20.0 | - | 10.0 | - |
| Rezal 36GP[3] | - | - | - | 20.0 | 15.0 | 25.0 |
| Ethanol (anhydrous) | 60.8 | - | 67.4 | 70.0 | - | 60.8 |
| Isopropanol (anhydrous) | - | 62.7 | - | - | 60.8 | - |
| Bentone 38 | 10.0 | 10.0 | 8.0 | 8.0 | - | 10.0 |
| Bentone 27 | - | - | - | - | 10.0 | - |
| Perfume | 1.0 | 1.0 | 1.0 | 1.0 | 1.0 | 1.0 |
| Urea | 3.2 | 1.3 | 3.6 | 1.0 | 3.2 | 3.2 |

%

| Examples: | 7 | 8 | 9 | 10 | 11 | 12 |
|---|---|---|---|---|---|---|
| AACH[1] | 25.0 | - | - | - | - | - |
| AACH/Urea complex[2] | - | 25.0 | 20.0 | - | - | - |
| ACH[4] | - | - | - | 25.0 | 25.0 | 20.0 |
| Ethanol (anhydrous) | 51.3 | 58.0 | 51.5 | 61.5 | 63.5 | 68.0 |
| VS 7207[5] | 10.0 | - | - | - | - | - |
| DPGME[6] | - | 8.0 | 18.0 | - | - | - |
| Bentone 38 | 10.0 | 7.0 | 9.0 | 10.0 | 10.0 | 8.0 |
| Perfume | 1.0 | 1.0 | 1.0 | 1.0 | 1.0 | 1.0 |
| Urea | 2.7 | 1.0 | 0.5 | 2.5 | - | - |
| Sodium hydroxide | - | - | - | - | 0.5 | - |
| Trisodium citrate | - | - | - | - | - | 3.0 |

1 - Spray-dried powder of a special active form of aluminium chloride prepared according to US Patent No.4 359 456. It had a particle size of less than 75 microns. It has a Band III percent Aluminium Value of greater than 20%.

2 - This is a pre-formed complex of urea and a special active form of aluminium chloride (AACH) prepared by codissolving AACH and urea (mole ratio 2:1) in water and spray drying the solution.

3 - Aluminium zirconium tetrachlorohydrate glycine complex available from Reheis Chemical Company. It has a particle size of less than 53 $\mu m$ (at least 98.5% less than 44 $\mu m$ ).

4 - Aluminium chlorhydrate available from Reheis Chemical Company under the trade mark MICRODRY.

5 - A cyclic polydimethylsiloxane mainly tetramer available from Union Carbide.

6 - Dipropylene glycol methyl ether available from Dow Chemical under the trade name Dowanol DPM.

The products of the Examples are made by shearing the suspending agent in the liquid component or mixture of the liquid components, excluding the perfume, until fully dispersed. The urea, sodium hydroxide and the trisodium citrate were added to the alcohol prior to the addition of the antiperspirant powder. The antiperspirant powder and perfume are then added and dispersed by further high shear mixing. The product is then filled into roll-on dispensers.

The products of Examples 1, 2 and 6 were assessed by the following test procedure involving subjecting human volunteers to thermal stress and gravimetric determination of axilla sweat.

Subjects:

A panel of up to 60 women who use no antiperspirant for the 14 days before the test.

Hot Room:

Temperatures 40°C± 2°C; relative humidity 40% ± 5%.

Products:

When testing two products, one being designated the test product and the other the control, the panel is divided into two equal groups. One group receives the test treatment on the left axilla and the control treatment on the right, while the second group receives them the other way round. Alternatively, when comparing two test products against each other and against a control product, then the products are randomly applied to the axillae of the panel subjects, with the proviso that the product applied to the left axilla is different from that applied to the right axilla of each subject.

Control Product:

This is a placebo deodorant in the form of an aerosol product comprising by weight 25% ethanol, 0.6%

EP 0 274 267 B1

isopropyl myristate, 0.3% perfume, and 74.1% propellant (1:1 mixture of Propellants 11 & 12).

Product Application:

The operator conducting the test applies the test product in the standard manner, so as to deposit an appropriate quantity of product, for example, on average about 300 mg of product on each axilla.

Sweat Collection:

Absorbent cotton pads are used to collect the sweat. On entering the hot room each panellist is subjected to a 40 minute 'warm-up' period, during which no sweat is collected. Sweat is then collected for a 20 minute period and sweat weight determined.

Test Design:

Subjects attend daily for 3 consecutive days. They receive one treatment with the products on each of the first three days. Following product application on the third day, the panellist is subjected to a hot room sitting and sweat is collected.

Analysis of Data:

The statistical treatment includes an analysis of variance which allows for side effects due to the product and the panellist. The efficacy is calculated from the geometric mean weight of sweat collected from the axillae treated with each product using the formula:

$$\% \text{ reduction} = 100 \frac{(C-T)}{C}$$

where C is the geometric mean sweat weight from the axillae treated with the control product and T is the geometric mean sweat weight from the axillae treated with the test product where a correction has been made for the side effect. Significance is calculated by applying Student's t-test to the logarithmically transformed weights.

The results of tests conducted with each of the products of Examples 1, 2 and 6 gave sweat reductions as indicated below.

| Product | % Sweat Reduction |
|---|---|
| Example 1 | 58 |
| Example 2 | 53 |
| Example 6 | 58 |

The products of all of Examples 1 to 12 are stable antiperspirant lotions which dry quickly to form a structured, tenacious film on the skin. The urea, sodium hydroxide or trisodium citrate present enhance the stability of the perfume, and reduce the solubility of the antiperspirant agent in the ethanol present, thus improving the efficacy of the product. They can each also improve the structure imparted by the Bentone and allow less of the suspending agent to be used. The DPGME present in Examples 8 and 9 results in a less white deposit on the skin.

Example 13

The following is the formulation of an antiperspirant aerosol product made according to the invention.

25

| | % |
|---|---|
| Aluminium chlorohydrate | 3.5 |
| Anhydrous ethanol | 9.5 |
| Bentone 27 | 0.5 |
| Propylene carbonate | 0.165 |
| Urea | 0.5 |
| Perfume | 0.2 |
| Propellant[1] | to 100.0 |

1 – mixture of 35 parts dichlorodifluoromethane and 65 parts trichloromonofluoromethane.

The Bentone 27 is sheared in the mixture of the liquid components, excluding the propellant and perfume, until fully dispersed. The urea is pre-dissolved in the alcohol. The aluminium chlorohydrate and perfume are then added. The resulting concentrate is then placed in a can, sealed with a suitable aerosol valve, and pressurised with the propellant.

Example 14

The following is the formulation of an antiperspirant stick made according to the invention.

| | % |
|---|---|
| Rezal 36GP | 22 |
| Anhydrous ethanol | 39 |
| Bentone 38 | 5 |
| Fluid AP[1] | 6 |
| Stearyl alcohol | 25 |
| Perfume | 1 |
| Urea | 2 |

1 –  A hydrophobic oil, a product of Union Carbide; its CTFA name is PPG-14 Butyl Ether.

The above ingredients, except for the perfume, were heated together with stirring at a temperature of 65°C for 10 minutes and then cooled to 50°C, at which point the perfume was added with continued stirring. The mixture so obtained was poured into stick moulds and allowed to cool and to set to form antiperspirant sticks.

**Claims**

1. An anhydrous antiperspirant product suitable for applying to the skin, which comprises:
   (i) from 1 to 60% by weight of a finely-divided Powder antiperspirant agent;
   (ii) from 10 to 90% by weight of an anhydrous liquid or solidified liquid medium, comprising an anhydrous alcohol chosen from ethanol, isopropanol or a mixture thereof;

(iii) from 0.001 to 10% by weight of a perfume; and

(iv) from 0.1 to 25% by weight, expressed in terms of the anhydrous alcohol present, of a perfume-stabilising agent chosen from compounds having a basic nitrogen function compounds having a basic oxygen function, drying agents or mixtures thereof.

2. An antiperspirant product according to Claim 1, in which the antiperspirant agent has a mean particle diameter of not greater than 100$\mu$m.

3. An antiperspirant product according to Claim 1 or 2, in which the antiperspirant agent is chosen from aluminium salts, zirconium salts or mixtures thereof.

4. An antiperspirant product according to any of Claims 1, 2 or 3, in which the antiperspirant agent is an aluminium chlorohydrate.

5. An antiperspirant product according to any preceding Claim, in which the antiperspirant agent is chosen from aluminium zirconium trichlorohydrex-GLY, aluminium zirconium tetrachlorohydrex-GLY or mixtures thereof.

6. An antiperspirant product according to any of the preceding claims, in which the perfume stabilizing agent has a basic nitrogen function and is chosen from urea, natural amino acids, organic amines, $C_1$ to $C_{20}$ alkylamines and hydroxylamines.

7. An antiperspirant product according to claim 6, in which the stabilizing agent is urea in an amount of from 0.5% to 25% of the alcohol present.

8. An antiperspirant product according to claim 6, wherein the perfume stabilizing agent is an amino acid and is either glycine or leucine, in an amount of from 0.5 to 25% by weight of the alcohol.

9. An antiperspirant product according to claim 6 wherein the perfume stabilizing agent is a $C_1$ to $C_{20}$ alkylamine or hydroxyalkyl amine, in an amount of from 0.5% to 4% by weight of the alcohol.

10. An antiperspirant product according to any of claims 1 to 5, in which the perfume stabilizing agent has a basic oxygen function and is chosen from inorganic bases, hydroxides of alkali metal and alkali-earth metals and ammonium hydroxide, basic metallic oxides, alkali metal salts of inorganic acids and $C_1$ to $C_{20}$ organic carboxylic acids, and mixtures thereof.

11. An antiperspirant product according to any of Claims 1 to 5, in which the perfume stabilising agent is a particulate alcohol-insoluble inorganic drying agent.

12. An antiperspirant product according to Claim 11, in which the drying agent is a molecular sieve.

13. An antiperspirant product according to any preceding claim, which additionally comprises a suspending agent chosen from hydrophobically-treated clays.

14. An antiperspirant product according to Claim 13, in which the clay is chosen from smectite clays, hormite clays or mixtures thereof.

15. An antiperspirant product according to Claims 13 or 14, in which the hydrophobically-treated clay is quaternium 18 hectorite.

16. A process for the manufacture of an anhydrous antiperspirant product suitable for applying to the skin, which process comprises the step of mixing together ingredients in amounts expressed in terms of the final product, namely:-

(i) from 1 to 60% by weight of a finely-divided powder antiperspirant agent;

(ii) from 10 to 90% by weight of an anhydrous liquid or solidified liquid medium, comprising an anhydrous alcohol chosen from ethanol, isopropanol or a mixture thereof;

(iii) from 0.001 to 10% by height of a perfume; and

(iv) from 0.1 to 25% y weight, expressed in terms of the anhydrous alcohol present, of a perfume-

EP 0 274 267 B1

stabilising agent chosen from compounds having a basic nitrogen function, compounds having a basic oxygen function, drying agents or mixtures thereof;
provided that the perfume stabilising agent is added to the anhydrous alcoholic ingredient either before or simultaneously with the perfume.

**Revendications**

1. Produit anti-perspirant anhydre qui convient pour application à la peau, qui comprend :
   (i) de 1 à 60% en poids d'un agent anti-perspirant pulvérulent finement divisé ;
   (ii) de 10 à 90% en poids d'un milieu liquide anhydre ou liquide solidifié comprenant un alcool anhydre choisi parmi l'éthanol, l'isopropanol ou un mélange de ceux-ci;
   (iii) de 0,001 à 10% en poids d'un parfum ; et
   (iv) de 0,1 à 25% en poids, exprimés en alcool anhydre présent d'un agent stabilisant de parfum choisi parmi les composés ayant une fonction d'azote basique, les composés ayant une fonction d'oxygène basique, les agents siccatifs ou leurs mélanges.

2. Produit anti-perspirant selon la revendication 1, dans lequel l'agent anti-perspirant présente une granulométrie moyenne ne dépassant pas 100 $\mu$m de diamètre.

3. Produit anti-perspirant selon la revendication 1 ou 2, dans lequel l'agent anti-perspirant est choiparmi les sels d'aluminium, les sels de zirconium ou les mélanges de ceux-ci.

4. Produit anti-perspirant selon l'une quelconque des revendications 1, 2 et 3, dans lequel l'agent antiperspirant est un chlorhydrate d'aluminium.

5. Produit anti-perspirant selon l'une quelconque des revendications précédentes, dans lequel l'agent anti-perspirant est choisi parmi le trichlorhydrex-GLY d'aluminium et de zirconium, le tetrachlorhydrex-GLY d'aluminium et de zirconium ou des mélanges de ceux-ci.

6. Produit anti-perspirant selon l'une quelconque des revendications précédentes, dans lequel l'agent stabilisant de parfum possède une fonction d'azote basique et est choisi parmi l'urée, les amino-acides naturels, les amines organiques, les alkylamines en $C_{1-20}$ et les hydroxylamines.

7. Produit anti-perspirant selon la revendication 6, dans lequel l'agent stabilisant est l'urée à raison de 0,5 à 25% de l'alcool présent.

8. Produit anti-perspirant selon la revendication 6, dans lequel l'agent stabilisant de parfum est un amino-acide et est la glycine ou la leucine, à raison de 0,5 à 25% en poids de l'alcool .

9. Produit anti-perspirant selon la revendication 6, dans lequel l'agent stabilisant de parfum est une alkylamine en $C_{1-20}$ ou une hydroxyalkylamine, à raison de 0,5 à 4% en poids de l'alcool.

10. Produit anti-perspirant selon l'une quelconque des revendications 1 à 5, dans lequel l'agent stabilisant de parfum possède une fonction d'oxygène basique et il est choisi parmi les bases minérales, les hydroxydes de métaux alcalins et de métaux alcalino-terreux et d'hydroxyde d'ammonium, les oxydes métalliques basiques, les sels de métaux alcalins d'acides minéraux et des acides carboxyliques organiques en $C_{1-20}$, ainsi que des mélanges de ceux-ci.

11. Produit anti-perspirant selon l'une quelconque des revendications 1 à 5, dans lequel l'agent stabilisant de parfum est un agent siccatif minéral particulaire insoluble dans l'alcool.

12. Produit anti-perspirant selon la revendication 11, dans lequel l'agent siccatif est un tamis moléculaire.

13. Produit anti-perspirant selon l'une quelconque des revendications précédentes, qui contient, en outre, un agent de suspension choisi parmi les argiles traitées par voie hydrophobe .

14. Produit anti-perspirant selon la revendication 13, dans lequel l'argile est choisie parmi les argiles smectiques, les argiles hormites ou des mélanges de celles-ci.

28

**15.** Produit anti-perspirant selon la revendication 13 ou 14, dans lequel l'argile traitée par voie hydrophobe est l'hectorite quaternium-18.

**16.** Procédé de fabrication d'un produit antiperspirant anhydre qui convient pour application à la peau, procédé qui consiste à mélanger ensemble les ingrédients en des proportions exprimées par rapport au produit final, à savoir :

(i) de 1 à 60% en poids d'un agent anti-perspirant pulvérulent finement divisé ;

(ii) de 10 à 90% en poids d'un milieu liquide anhydre ou liquide solidifié comprenant un alcool anhydre choisi parmi l'éthanol, l'isopropanol ou un mélange de ceux-ci;

(iii) de 0,001 à 10% en poids d'un parfum ; et

(iv) de 0,1 à 25% en poids, exprimés en alcool anhydre présent d'un agent stabilisant de parfum choisi parmi les composés ayant une fonction d'azote basique, les composés ayant une fonction d'oxygène basique, les agents siccatifs ou leurs mélanges ;

à la condition que l'agent stabilisant de parfum soit ajouté aux ingédients alcooliques anhydres avant ou en même temps que le parfum.

**Patentansprüche**

**1.** Wasserfreies Antiperspirant-Produkt, das zum Auftragen auf die Haut geeignet ist, umfassend:

(i) 1 bis 60 Gew.-% eines feinzerteilten Antiperspirant-Mittels in Pulverform;

(ii) 10 bis 90 Gew.-% einer wasserfreien Flüssigkeit oder festgewordenen flüssigen Mittels, umfassend einen wasserfreien Alkohol, ausgewählt unter Ethanol, Isopropanol oder einer Mischung davon;

(iii) 0,001 bis 10 Gew.-% eines Parfums und

(iv) 0,1 bis 25 Gew.-%, bezogen auf den anwesenden wasserfreien Alkohol, eines Parfum-stabilisierenden Mittels, ausgewählt unter Verbindungen, die eine basische Stickstoff-Funktion aufweisen, Verbindungen, die eine basische Sauerstoff-Funktion aufweisen, Trocknungsmitteln oder Mischungen davon.

**2.** Antiperspirant-Produkt nach Anspruch 1, worin das Antiperspirant-Mittel einen mittleren Teilchendurchmesser von nicht größer als 100 $\mu$m aufweist.

**3.** Antiperspirant-Produkt nach Anspruch 1 oder 2, worin das Antiperspirant-Mittel ausgewählt ist unter Aluminiumsalzen, Zirkoniumsalzen oder Mischungen davon.

**4.** Antiperspirant-Produkte nach einem der Ansprüche 1, 2 oder 3, worin das Antiperspirant-Mittel ein Aluminiumchlorhydrat ist.

**5.** Antiperspirant-Produkt nach einem der vorhergehenden Ansprüche, worin das Antiperspirant-Mittel ausgewählt ist unter Aluminium-Zirkonium-trichlorhydrex-GLY, Aluminium-Zirkonium-tetrachlorhydrex-GLY oder Mischungen davon.

**6.** Antiperspirant-Produkt nach einem der vorhergehenden Ansprüche, worin das Parfum-Stabilisierungsmittel eine basische Stickstoff-Funktion hat und ausgewählt ist unter Harnstoff, natürlichen Aminosäuren, organischen Aminen, $C_1$ bis $C_{20}$ Alkylaminen und Hydroxylaminen.

**7.** Antiperspirant-Produkt nach Anspruch 6, worin das Stabilisierungsmittel Harnstoff ist, in einer Menge von 0,5% bis 25% des anwesenden Alkohols.

**8.** Antiperspirant-Produkte nach Anspruch 6, worin das Parfum-Stabilisierungsmittel eine Aminosäure und entweder Glycin oder Leucin ist, in einer Menge von 0,5 bis 25 Gew.-% des Alkohols.

**9.** Antiperspirant-Produkte nach Anspruch 6, worin das Parfum-Stabilisierungsmittel ein $C_1$ bis $C_{20}$ Alkylamin oder Hydroxyalkylamin ist, in einer Menge von 0,5 bis 4 Gew.-% des Alkohols.

**10.** Antiperspirant-Produkt nach einem der Ansprüche 1 bis 5, worin das Parfum-Stabilisierungsmittel eine basische Sauerstoff-Funktion hat und ausgewählt ist unter anorganischen Basen, Hydroxiden von Alkalimetallen und Erdalkalimetallen und Ammoniumhydroxid, basischen Metalloxiden, Alkalimetallsalzen von anorganischen Säuren und $C_1$ bis $C_{20}$ organischen Karbonsäuren und Mischungen davon.

**11.** Antiperspirant-Produkt nach einem der Ansprüche 1 bis 5, worin das Parfum-Stabilisierungsmittel ein teilchenförmiges, Alkohol-unlösliches, anorganisches Trocknungsmittel ist.

**12.** Antiperspirant-Produkt nach Anspruch 11, worin das Trocknungsmittel ein Molekularsieb ist.

**13.** Antiperspirant-Produkt nach einem der vorhergehenden Ansprüche, das zusätzlich ein Suspendierungsmittel, ausgewählt unter hydrophob-behandelten Tonen, umfaßt.

**14.** Antiperspirant-Produkt nach Anspruch 13, worin der Ton ausgewählt ist unter Smektit-Tonen, Hormit-Tonen oder Mischungen davon.

**15.** Antiperspirant-Produkt nach Anspruch 13 oder 14, worin der hydrophob-behandelte Ton Quaternium-18-Hektorit ist.

**16.** Verfahren zur Herstellung eines wasserfreien Antiperspirant-Produkts, das zum Auftragen auf die Haut geeignet ist, wobei das Verfahren den Schritt des Zusammenmischens von Bestandteilen umfaßt, in Mengen bezogen auf das Endprodukt, nämlich:
(i) 1 bis 60 Gew.-% eines feinzerteilten Antiperspirant-Mittels in Pulverform;
(ii) 10 bis 90 Gew.-% einer wasserfreien Flüssigkeit oder festgewordenen flüssigen Mittels, umfassend einen wasserfreien Alkohol, ausgewählt unter Ethanol, Isopropanol oder einer Mischung davon;
(iii) 0,001 bis 10 Gew.-% eines Parfums und
(iv) 0,1 bis 25 Gew.-%, bezogen auf den anwesenden wasserfreien Alkohol, eines Parfum-stabilisierenden Mittels, ausgewählt unter Verbindungen, die eine basische Stickstoff-Funktion aufweisen, Verbindungen, die eine basische Sauerstoff-Funktion aufweisen, Trocknungsmitteln oder Mischungen davon,
vorausgesetzt, daß das Parfum-Stabilisierungsmittel zu dem wasserfreien alkoholischen Bestandteil entweder vor oder gleichzeitig mit dem Parfum zugefügt wird.